# EUROPEAN PATENT SPECIFICATION

(11) **EP 1 817 022 B1**
(45) Date of publication and mention of the grant of the patent: **25.04.2018**
(21) Application number: 05852217.8
(22) Date of filing: 23.11.2005
(51) Int. Cl.: A61K 31/385, A61K 31/33, A61P 37/04

(54) **METHODS FOR INCREASING THE IMMUNE RESPONSE IN AN ANIMAL**
VERFAHREN ZUR STEIGERUNG DER IMMUNANTWORT BEI EINEM TIER
PROCEDES DESTINES A ACCROITRE LA REPONSE IMMUNITAIRE CHEZ UN ANIMAL

(30) Priority: 24.11.2004 US 630972 P
(43) Date of publication of application: 15.08.2007
(73) Proprietor: Hill's Pet Nutrition, Inc., Topeka, KS 66603 (US)
(72) Inventor: PAETAU-ROBINSON, Inke, Auburn, Kansas 66402 (US); ZICKER, Steven Curtis, Lawrence, Kansas 66049 (US)
(74) Representative: Wichmann, Hendrik
(86) International application number: PCT/US2005/042805
(87) International publication number: WO 2006/058248

(56) References cited:
- WO-A-01/00194
- WO-A-02/47703
- WO-A-02/096414
- WO-A-03/035056
- WO-A-2006/058278
- US-A1- 2005 232 976
- US-B1- 6 746 678
- US-B1- 6 974 841
- US-B2- 6 902 739
- US-B2- 6 914 071
- KöHLER H.B.K. ET AL.: 'Involvement of Reactive Oxygen Species in TNF-alpha mediated Activation of the Transcription Factor NF-kappaB in Canine Dermal Fibroblasts' VETERINARY IMMUNOLOGY AND IMMUNOPATHOLOGY vol. 71, no. 2, 1999, pages 125 - 142, XP003010211

## Description

### BACKGROUND OF THE INVENTION

### Field of the Invention

The present invention generally relates to lipoic acid for use in enhancing the immune response in an animal, wherein the use comprises increasing natural killer cell activity in an animal, wherein the lipoic acid is provided in a composition, and wherein the animal is susceptible to or suffering from impaired immune response and is in need of increased or improved immune response.

### Description of the Related Art

Due to the constant exposure to an incredible diversity of bacteria, viruses and parasites, most mammals have developed an immune system to defend against such attacks. Natural Killer (NK) cells are important effectors of the innate immune response that have been described on a functional basis as capable of killing tumors or virally infected cells without previous stimulation. Studies have shown that as some mammals age their immune response degrades. The health of their companion animal such as a dog is very important to many people, especially as the animal ages. What is needed is a method to increase NK cell activity in animals, particularly in aging animals.
WO 01/00194 discloses compositions for optimizing immune activity in the treatment of auto-immune diseases and chronic immune conditions. An immune enhancing effect of lipoic acid is not disclosed.

### SUMMARY OF THE INVENTION

The present invention provides lipoic acid for use in enhancing the immune response in an animal, wherein the use comprises increasing natural killer cell activity in an animal, wherein the lipoic acid is provided in a composition, and wherein the animal is susceptible to or suffering from impaired immune response and is in need of increased or improved immune response. Further preferred embodiments of the invention are defined in the claims.

In various embodiments, the invention is a new approach for improving the health of aging animals based upon the use of lipoic acid as part of a diet that is fed to the animals. The new approach involves increasing natural killer cell activity in animals, especially dogs, based upon the use of lipoic acid as part of a diet that is fed to the animals.

Further areas of applicability of the present invention will become apparent from the detailed description provided hereinafter. It should be understood that the detailed description and specific examples, while indicating illustrative embodiments of the invention, are not intended to limit the scope of the invention, which is only defined by the claims.

### BRIEF DESCRIPTION OF THE DRAWINGS

The present invention will become more fully understood from the detailed description and the accompanying drawings.

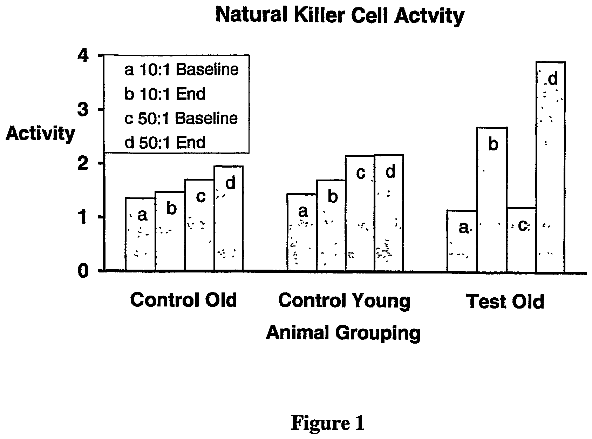

Figure 1 is a graphical representation of the effect of lipoic acid on NK cell activity in old dogs as compared to young and old controls.

The Figure is intended to exemplify the general characteristics of the invention for the purposes of the description of such embodiments herein. This Figure may not precisely reflect the characteristics of any given embodiment and is not necessarily intended to define or limit specific embodiments within the scope of this invention, which is only defined by the claims.

### DETAILED DESCRIPTION OF THE INVENTION

### Definitions

The term "animal" means any animal susceptible to or suffering from impaired immune response and in need of increased or improved immune response or an animal that could benefit from an increased immune response. An animal is "susceptible to" a disease or condition if the animal exhibits symptoms that indicate that the animal is likely to develop the condition or disease. An animal is "suffering from" a disease or condition if the animal exhibits symptoms that are indicative that the anima has developed the condition or disease.

The term "older animal" means any animal susceptible to or suffering from impaired immune response and in need of increased or improved immune response or an animal that could benefit from an improved immune response because of age.

The term "single package" means that the components of a kit are physically associated in or with one or more containers and considered a unit for manufacture, distribution, sale, or use. Containers include, but are not limited to, bags, boxes, bottles, shrink wrap packages, stapled or otherwise affixed components, or combinations thereof. A single package may be containers of individual food compositions physically associated such that they are considered a unit for manufacture, distribution, sale, or use.

The term "virtual package" means that the components of a kit are associated by directions on one or more physical or virtual kit components instructing the user how to obtain the other components, e.g., in a bag containing one component and directions instructing the user to go to a website, contact a recorded message, view a visual message, or contact a caregiver or instructor to obtain instructions on how to use the kit.

### The Invention

In one aspect, the present invention provides lipoic acid for use in enhancing the immune response in an animal, wherein the use comprises increasing natural killer cell activity in an animal, wherein the lipoic acid is provided in a composition, and wherein the animal is susceptible to or suffering from impaired immune response and is in need of increased or improved immune response. Generally, the lipoic acid is fed to the animal in a composition in amounts of greater than 5 mg per day, preferably from about 10 to about 1000 mg per day, most preferably from about 50 to about 500 mg per day.

The compositions comprise lipoic acid in amounts of at least 50 ppm, preferably at least 150 ppm. In various embodiments, the compositions are useful for increasing immune function and response in animals, particularly in older animals, as defined in the claims.

The nutrition and health of companion animals is one of the most important aspects of care, particularly pet care for animals. Many caregivers have a difficult time determining if an animal is receiving a well-balanced and healthy diet. While people are becoming much more aware regarding their own personal nutrition, there is little knowledge of the advanced dietary requirements that an animal must have. Natural Killer (NK) cells are important effectors of the innate immune response that have been described on a functional basis as capable of killing tumors or virally infected cells without previous stimulation. Studies have shown that as some mammals age their immune response degrades. Since the immune response is degrading, an increase in NK cell activity may improve the health of the aging mammal.
The present invention provides a composition or diet containing lipoic acid for use in enhancing the immune response in an animal, e.g. a companion animal such as a dog, particularly when it may be impaired by age.

The amount of lipoic acid given to the animal is a non-toxic amount. The lipoic acid may be provided to the animal either as a supplement or contained in a composition, including a diet, fed to the animal. Such a supplement may be in the form of a pill or capsule, a treat or a biscuit, or any other edible form. By "diet", it is meant the food or drink regularly consumed by the animal. A diet may include supplements consumed by the animal. A diet is considered to have essentially enough nutrients to be life sustaining for the animal. A companion animal diet can be any suitable pet food formula which provides adequate nutrition for the animal. For example, a typical canine diet for use in the present invention may contain from about 8 to 50% fat, about 16 to 50% by weight protein and about 3 to 15% total dietary fiber. In another example, a typical feline diet may contain about 8 to 50% by weight fat and from about 30 to 60% by weight protein. However, no specific ratios or percentages of these or other nutrients are required. A nutrient is any food constituent that helps support life. Nutrients important to an animal's health are known to skilled artisans, e.g., proteins, carbohydrates, fats, fibers, vitamins, and minerals. Water is also vital to an animal's health.

The free radical theory of aging proposes that oxidative stress results in aging and a decrease in the reduced to oxidized ratio of intracellular anti-toxins such as glutathione. Glutathione is prevalent in the liver and is utilized to conjugate xenobiotics for the elimination through the bile duct and eventual elimination via the feces. As such, decreased concentration and activity of glutathione in aged animals may result in the impaired clearance of xenobiotics that contribute to cancer, toxicity and other unwanted effects. In addition, it is known that immune function undergoes senescence with increasing age. Previous studies have shown that GSH:GSSG ratios may be improved in lymphocytes from dogs supplemented with lipoic acid, however, a functional outcome has not been quantified. (See Zicker, SC et al., Veterinary Therapeutics, 3(2):167-176, 2002). Attempts have been made to improve immune response in companion animals by introducing beta-carotene in combination with other antioxidants. (See U.S. Patent No. 6,310,090 to Hayek issued October 30, 2001 and U.S. Patent No. 6,133,323 to Hayek issued October 17, 2000).

R-α-lipoic acid (CAS number 1200-22-2, also known as thioctic acid and 1, 2-dithiolane-3-pentanoic acid) naturally occurs in plant and animal tissues, where it is covalently bound to an ε-amino group of lysine residues. Lipoic acid is commercially available and is produced by companies such as BASF and Cognis. Lipoic acid is commercially available as essentially pure R-α lipoic acid or as racemic mixtures of lipoic acid isomers. In plants, lipoic acid is most abundant in spinach and potatoes while in animal tissues, lipoic acid is most abundant in the kidney and the heart. R-α-lipoic acid was first discovered in 1937 (See Snell et al., Journal Bact. 33; 207, 1937) and was not isolated and characterized until 1951 (See Reed et al. Science 114:94-4, 1951). R-α-lipoic acid may be synthesized and such methods are well known in the art. (See U.S. Patent No. 2,890,716 to Reed issued April 18, 1961). R-α-lipoic acid has been classified as an antioxidant and has been used in high dosages as a treatment for Type II diabetes. Studies have shown that mixtures of carnitine and lipoic acid may enhance metabolism and alleviate oxidative stress. (See U.S. Patent No. 5,916,912 to Ames et al. issued June 29, 1999 and U.S. Patent No. 6,365,622 to Cavayzo issued April 2, 2002). In addition, it has been shown that a companion animal diet comprising lipoic acid among other ingredients appears to inhibit the deterioration of the mental capacity of an aged companion animal. (See U.S. Patent Application Nos. 20020076469, 20020052402, 20020076470, 2000115710, and 20020119182.)

NK cells show antibody-independent cell mediation cytotoxicity against target cells such as virus infected cells and tumor cells. In humans, mice and rats, it has been suggested that NK cells recognize and bind to a target cell and then release cytotoxic factors to destroy the target cell. Such binding and cytotoxic behavior of NK cells have been reported in canines (see Nariai, Y. et al., J. Bte. Med. Sci. 61 (7): 835-838, 1999), although the mechanism differs from the above animal examples. Studies have shown that mitochondrial oxidation plays a role in the metabolism of lipoic acid. Although the metabolism in humans mainly resembles that observed in mice and rats, the formation of oxidized structures related to tetranorlipoic acid found in canines appears to have no equivalent in humans. In addition, 3-ketolipoic acid, an intermediate in the mitochondrial oxidation of lipoic acid has been reported in plasma samples from rats and humans but has not been found in plasma from canines. (See Schupke, H. et al. Drug Metabolism and Disposition, 29 (6) 855-862, 2001). It appears that the metabolic pathway of α-lipoic acid is different in canines as compared to humans.

Various embodiments of the invention include
feeding to the animal a composition, e.g., a diet, comprising lipoic acid in an amount of at least 50 ppm on a dry matter basis per day. Other embodiments comprises feeding to the animal a diet comprising lipoic acid in an amount of at least 100 ppm on a dry matter basis per day. Still other embodiments comprises feeding to the animal a diet comprising lipoic acid in an amount from about 75 ppm to about 150 ppm on a dry matter basis per day. As used herein, lipoic acid is in a racemic mixture, but other embodiments of the invention may include lipoic acid which is essentially pure R-α lipoic acid or as a lipoate derivative, mixtures of isomers, salts, esters, amides or combinations thereof (For example see US Patent No 5,621,177 to Bethge et al. issued April 15, 1997).

In various embodiments of the invention, a companion pet composition or diet comprising at least 50 ppm of lipoic acid, dry matter basis, is used for increasing the immune response in older dogs. In some embodiments of the invention, the lipoic acid is added to the companion animal's food. In such embodiments, the lipoic acid may be added during the processing of the companion animal food that is then packaged and made available to consumers. Such processes may include extrusion, canning, baking, and the like or any other method or process of producing pet foods that is known in the art. In such processes, the lipoic acid may be contributed by a natural source like an animal or plant component, such as kidney or spinach, or the lipoic acid may be contributed by a synthetically derived source, or the lipoic acid may be contributed by a mixture of natural and synthetic sources. In other embodiments of the invention, lipoic acid may be in a capsule form to be fed to the companion animal. In still other embodiments of the invention, the lipoic acid may be in a powder or in a crystalline which may be added to the animal's food or fed directly to the animal. In various embodiments of the invention, the companion animal diet comprises lipoic acid and other needed nutritional components. In various embodiments of the invention, the companion animal is a dog and in other embodiments of the invention, the companion animal is a cat. Studies have shown that lipoic acid may be ten times more toxic in cats than in dogs. (See Hill, AS et al., J. Anim. Physiol. Anim. Nutr. 88(3-4): 150-156, 2004). In various embodiments of the invention wherein the companion animal is a cat, the diet comprises less than 30 ppm of lipoic acid on a dry weight basis,

In a further aspect, the invention provides for the use of lipoic acid to prepare a medicament for increasing natural killer cell activity in an animal by feeding a natural killer cell activity increasing amount of lipoic acid to the animal, as defined in the claims. Generally, medicaments are prepared by admixing a compound or composition with excipients, buffers, binders, plasticizers, colorants, diluents, compressing agents, lubricants, flavorants, moistening agents, and other ingredients known to skilled artisans to be useful for producing medicaments and formulating medicaments that are suitable for administration to an animal.

Also disclosed are kits suitable for feeding lipoic acid to an animal. The kits comprise in separate containers in a single package or in separate containers in a virtual package, as appropriate, lipoic acid and at least one of (1) one or more ingredients suitable for consumption by an animal, (2) instructions for how to combine the lipoic acid and other kit components to increase immune response, particularly to produce a composition useful for increasing immune response, and (3) instructions for how to use the lipoic acid and other components of the present invention, particularly for the benefit of the animal. When the kit comprises a virtual package, the kit is limited to instructions in a virtual environment in combination with one or more physical kit components. The kit contains the lipoic acid and other components in amounts sufficient to increase immune response. Typically, the lipoic acid and the other suitable kit components are admixed just prior to consumption by an animal, In one embodiment, the kit contains a packet containing lipoic acid and a container of food for consumption by an animal. The kit may contain additional items such as a device for mixing the lipoic acid and ingredients or a device for containing the admixture, e.g., a food bowl. In another embodiment, the lipoic acid is mixed with additional nutritional supplements such as vitamins and minerals that promote good health in an animal.

This invention is not limited to the particular methodology, protocols, and reagents described herein because they may vary. Further, the terminology used herein is for the purpose of describing particular embodiments only and is not intended to limit the scope of the present invention. As used herein and in the appended claims, the singular forms "a," "an," and "the" include plural reference unless the context clearly dictates otherwise. The terms "comprise", "comprises", and "comprising" are to be interpreted inclusively rather than exclusively.

Unless defined otherwise, all technical and scientific terms and any acronyms used herein have the same meanings as commonly understood by one of ordinary skill in the art in the field of the invention. Although any methods and materials similar or equivalent to those described herein can be used in the practice of the present invention, the preferred methods, devices, and materials are described herein.

### EXAMPLES

### Example 1

The study involves three groups of dogs: Group 1 - young dogs on a controlled food, Group 2 - old dogs on a controlled food, and Group 3 - old dogs on a dry food fortified with 150 ppm of lipoic acid on a dry matter basis. The dogs are beagles and Group 1 consists of 10 beagles with the average age of 5.1 years old, Group 2 consists of 10 beagles with an average age of 11.8 years old, and Group 3 consists of 10 beagles with an average age of 11.3 years old. The dogs from all three groups are fed the controlled food for a two week period prior to intervention. After a two week period, Group 3 is transferred to a diet of dry food fortified with 150 ppm of lipoic acid on a dry matter basis. During the two week period on the control, samples are taken from all dogs and chemical and biological analysis is completed on the samples to establish base line data. Then three groups of dogs eat their respective diets for a six week period of time and after this time period, samples are taken from all of the dogs in the study and chemical and biological analysis is completed on the samples. Baseline data is compared to the data taken at the end of the six week study. Results as in Figure 1 show the dogs in Group 3 which included a diet with 150 ppm of lipoic acid on a dry matter basis have increased NK cell activity.

In the specification, there have been disclosed typical preferred embodiments of the invention and, although specific terms are employed, they are used in a generic and descriptive sense only and not for purposes of limitation, the scope of the invention being set forth in the following claims.

## Claims

1. Lipoic acid for use in enhancing the immune response of an animal,
wherein the use comprises increasing natural killer cell activity in the animal,
wherein the lipoic acid is provided in a composition, and
wherein the animal is susceptible to or suffering from impaired immune response and is in need of increased or improved immune response.

2. Lipoic acid for use according to claim 1 wherein said use comprises feeding the lipoic acid in an amount of greater than 50 mg per day.

3. Lipoic acid for use according to claim 1 wherein said use comprises feeding the lipoic acid in an amount of from about 10 to about 1000 mg per day.

4. Lipoic acid for use according to any preceding claim wherein the animal is a companion animal.

5. Lipoic acid for use according to any preceding claim wherein the companion animal is a canine.

6. Lipoic acid for use according to any preceding claim wherein the lipoic acid is in capsule form.

7. Lipoic acid for use according to any of claims 1 to 5 wherein the lipoic acid is in powder form.

8. Lipoic acid for use according to any of claims 1 to 5 wherein the lipoic acid is in crystalline form.

9. Lipoic acid for use according to any preceding claim wherein the lipoic acid is part of the animal's daily diet.

10. Lipoic acid for use according to any preceding claim wherein the lipoic acid is in the composition in an amount of greater than 50 ppm on a dry matter basis.

11. Lipoic acid for use according to any preceding claim wherein the animal is an older animal.

12. Lipoic acid for use according to any preceding claim wherein the lipoic acid is in a composition comprising:
a life sustaining amount of nutrients; and
greater than 50 ppm of lipoic acid.

13. Lipoic acid for use according to claim 12 wherein the composition is a food composition.

14. Lipoic acid for use according to claim 13 wherein the food composition is suitable for a companion animal.

15. Lipoic acid for use according to claim 13 or claim 14 wherein the food composition is suitable for a canine.

16. Lipoic acid for use according to any of claims 13 to 15 wherein the food composition is extruded or canned.

17. Use of lipoic acid for the preparation of a medicament for enhancing the immune response of an animal, wherein the lipoic acid increases natural killer cell activity in the animal, and
wherein the animal is susceptible to or suffering from impaired immune response and is in need of increased or improved immune response.

## Patentansprüche

1. Liponsäure zur Verwendung beim Verstärken der Immunantwort eines Tieres,
wobei die Verwendung Erhöhen der natürlichen Killerzellaktivität in dem Tier umfasst,
wobei die Liponsäure in einer Zusammensetzung bereitgestellt wird, und
wobei das Tier gegenüber beeinträchtigter Immunantwort empfänglich ist oder daran leidet, und erhöhte oder verbesserte Immunantwort benötigt.

2. Liponsäure zur Verwendung nach Anspruch 1, wobei die Verwendung Füttern der Liponsäure in einer Menge von größer als 50 mg pro Tag umfasst.

3. Liponsäure zur Verwendung nach Anspruch 1, wobei die Verwendung Füttern der Liponsäure in einer Menge von etwa 10 bis etwa 1000 mg pro Tag umfasst.

4. Liponsäure zur Verwendung nach einem beliebigen voranstehenden Anspruch, wobei das Tier ein Haustier ist.

5. Liponsäure zur Verwendung nach einem beliebigen voranstehenden Anspruch, wobei das Haustier ein Hund ist.

6. Liponsäure zur Verwendung nach einem beliebigen voranstehenden Anspruch, wobei die Liponsäure in Kapselform ist.

7. Liponsäure zur Verwendung nach einem beliebigen der Ansprüche 1 bis 5, wobei die Liponsäure in Pulverform ist.

8. Liponsäure zur Verwendung nach einem beliebigen der Ansprüche 1 bis 5, wobei die Liponsäure in kristalliner Form ist.

9. Liponsäure zur Verwendung nach einem beliebigen voranstehenden Anspruch, wobei die Liponsäure ein Teil der täglichen Ernährung des Tieres ist.

10. Liponsäure zur Verwendung nach einem beliebigen voranstehenden Anspruch, wobei die Liponsäure in der Zusammensetzung in einer Menge von größer als 50 ppm auf einer Trockenmassebasis vorliegt.

11. Liponsäure zur Verwendung nach einem beliebigen voranstehenden Anspruch, wobei das Tier ein älteres Tier ist.

12. Liponsäure zur Verwendung nach einem beliebigen voranstehenden Anspruch, wobei die Liponsäure in einer Zusammensetzung vorliegt, die umfasst:
eine lebenserhaltende Menge an Nährstoffen; und
größer als 50 ppm Liponsäure.

13. Liponsäure zur Verwendung nach Anspruch 12, wobei die Zusammensetzung eine Nahrungsmittelzusammensetzung ist.

14. Liponsäure zur Verwendung nach Anspruch 13, wobei die Nahrungsmittelzusammensetzung für ein Haustier geeignet ist.

15. Liponsäure zur Verwendung nach Anspruch 13 oder Anspruch 14, wobei die Nahrungsmittelzusammensetzung für einen Hund geeignet ist.

16. Liponsäure zur Verwendung nach einem beliebigen der Ansprüche 13 bis 15, wobei die Nahrungsmittelzusammensetzung extrudiert oder in Konservendosen abgefüllt ist.

17. Verwendung von Liponsäure zur Herstellung eines Medikaments zum Verstärken der Immunantwort eines Tieres, wobei die Liponsäure natürliche Killerzellaktivität in dem Tier erhöht, und
wobei das Tier gegenüber beeinträchtigter Immunantwort empfänglich ist oder daran leidet, und erhöhte oder verbesserte Immunantwort benötigt.

## Revendications

1. Acide lipoïque pour une utilisation dans l'amélioration de la réponse immunitaire d'un animal,
dans lequel l'utilisation comprend l'augmentation de l'activité des cellules tueuses naturelles chez l'animal,
dans lequel l'acide lipoïque est fourni dans une composition, et
dans lequel l'animal est prédisposé à ou souffre d'une réponse immunitaire altérée et a besoin d'une réponse immunitaire accrue ou améliorée.

2. Acide lipoïque pour une utilisation selon la revendication 1, dans lequel ladite utilisation comprend l'alimentation de l'acide lipoïque en une quantité supérieure à 50 mg par jour.

3. Acide lipoïque pour une utilisation selon la revendication 1, dans lequel ladite utilisation comprend l'alimentation de l'acide lipoïque en une quantité d'environ 10 à environ 1 000 mg par jour.

4. Acide lipoïque pour une utilisation selon l'une quelconque des revendications précédentes, dans lequel l'animal est un animal de compagnie.

5. Acide lipoïque pour une utilisation selon l'une quelconque des revendications précédentes, dans lequel l'animal de compagnie est un animal canin.

6. Acide lipoïque pour une utilisation selon l'une quelconque des revendications précédentes, dans lequel l'acide lipoïque est sous forme de gélule.

7. Acide lipoïque pour une utilisation selon l'une quelconque des revendications 1 à 5, dans lequel l'acide lipoïque est sous forme de poudre.

8. Acide lipoïque pour une utilisation selon l'une quelconque des revendications 1 à 5, dans lequel l'acide lipoïque est sous forme cristalline.

9. Acide lipoïque pour une utilisation selon l'une quelconque des revendications précédentes, dans lequel l'acide lipoïque fait partie de l'alimentation quotidienne de l'animal.

10. Acide lipoïque pour une utilisation selon l'une quelconque des revendications précédentes, dans lequel l'acide lipoïque est présent dans la composition en une quantité supérieure à 50 ppm sur une base de matière sèche.

11. Acide lipoïque pour une utilisation selon l'une quelconque des revendications précédentes, dans lequel l'animal est un animal âgé.

12. Acide lipoïque pour une utilisation selon l'une quelconque des revendications précédentes, dans lequel l'acide lipoïque est dans une composition comprenant :
une quantité de nutriments essentielle au maintien de la vie ; et
plus de 50 ppm d'acide lipoïque.

13. Acide lipoïque pour une utilisation selon la revendication 12, dans lequel la composition est une composition alimentaire.

14. Acide lipoïque pour une utilisation selon la revendication 13, dans lequel la composition alimentaire est appropriée pour un animal de compagnie.

15. Acide lipoïque pour une utilisation selon la revendication 13 ou la revendication 14, dans lequel la composition alimentaire est appropriée pour un animal canin.

16. Acide lipoïque pour une utilisation selon l'une quelconque des revendications 13 à 15, dans lequel la composition alimentaire est extrudée ou en conserve.

17. Utilisation d'acide lipoïque pour la préparation d'un médicament destiné à améliorer la réponse immunitaire d'un animal, dans laquelle l'acide lipoïque augmente l'activité des cellules tueuses naturelles chez l'animal, et
dans laquelle l'animal est prédisposé à ou souffre d'une réponse immunitaire altérée et a besoin d'une réponse immunitaire accrue ou améliorée.
